# EUROPEAN PATENT APPLICATION

(11) **EP 4 674 346 A1**
(43) Date of publication of application: **07.01.2026**
(21) Application number: 24185685.5
(22) Date of filing: 01.07.2024
(51) Int. Cl.: A61B 5/1455, A61B 5/00

(54) **PHOTOPLETHYSMOGRAPHY SENSOR**

(71) Applicant: Koninklijke Philips N.V., 5656 AG Eindhoven (NL)
(72) Inventor: PAULUSSEN, Elvira Johanna Maria, Eindhoven (NL); DAMINK, Paul, Eindhoven (NL); VAN DEN DUNGEN, Wilhelmus Andreas Marinus Arnoldus Maria, 5656AG Eindhoven (NL)
(74) Representative: Philips Intellectual Property & Standards

(57) **Abstract**

Proposed concepts thus aim to provide methods and systems pertaining to a PPG sensor system for measuring oxygen saturation of blood within bodily tissues. In particular, embodiments aim to provide a PPG sensor system configured for bidirectional optical radiation sensing by the configuration of first and second optical source arrangements and first and second optical detector arrangements.

## Description

### FIELD OF THE INVENTION

This invention relates to the field of Photoplethysmography (PPG) sensors and more particularly to PPG sensor systems for measuring oxygen saturation.

### BACKGROUND OF THE INVENTION

A PPG measurement refers to the use of optical measurements to detect blood volume changes in tissue. PPG measurements may be used to obtain measurements of vital signs such as such as measurements of heart rate, respiration rate, and blood oxygen saturation (SpO₂). PPG sensors therefore typically involve the emission of optical radiation to a bodily tissue and the detection of this optical radiation after it has interacted with the bodily tissue.

A pulse oximeter sensor is a type of PPG sensor that is used to measure the oxygen saturation level in a patient's blood. In pulse oximeter sensors, optical radiation travels through the tissue and is then detected by a photodiode. The optical radiation that travels through the tissue can be categorized into functional radiation and non-functional radiation. Functional radiation is optical radiation that contains pulsatile information from blood volume changes in the cardiac cycle. Non-functional radiation, does not contain pulsatile information and can introduce calibration artifacts in pulse oximeter sensors. This may include shunt radiation (radiation that did not pass through the tissue) and DC radiation that does pass through the tissue but doesn't contain any pulsatile information.

PPG sensors that are used for pulse oximetry typically come in two forms: transmission-based sensors, and reflection-based sensors. Transmission-based sensors measure optical radiation that has been transmitted through the full thickness of the tissue (for example, by placing an optical detector on the opposite side of the tissue to an optical detector), whereas reflection-based sensors measure optical radiation that is internally scattered/reflected within the patient's tissue and therefore does not traverse the tissue's full thickness (for example, by placing an optical detector on the same side of the tissue as an optical source). When using transmission-based PPG sensor systems for larger thickness tissues, calibration errors may occur. Transmissive ray paths through tissue may vary significantly depending on the wavelength of the radiation, the presence of moles, birthmarks or other spots on the tissue surface, and inhomogeneity within the tissue itself due to the non-uniformity of the position of blood vessels within the tissue. This variation affects the size and shape of the region of the tissue associated with functional radiation measured by the sensor. Inconsistency in the volume of this region depending on the properties of the bodily tissue being measured and the radiation being used can lead to calibration errors and thereby introduce inaccuracies and/or bias in the measured values of oxygen saturation.

### SUMMARY OF THE INVENTION

The invention is defined by the claims.

According to examples in accordance with an aspect of the invention, there is provided a PPG sensor for measuring oxygen saturation of blood within bodily tissue.

The system comprises: a support structure configured, in use, to receive bodily tissue; a first optical source arrangement coupled to the support structure and configured to emit optical radiation; a second optical source arrangement coupled to the support structure and configured to emit optical radiation; a first optical detector arrangement coupled to the support structure and configured to detect optical radiation incident on the first optical detector arrangement; and a second optical detector arrangement coupled to the support structure and configured to detect optical radiation incident on the second optical detector arrangement, wherein the support structure is configured such that, in use, the first optical source arrangement and the second optical detector arrangement are positioned at a first side of the bodily tissue, and the second optical source arrangement and the first optical detector arrangement are positioned at a second opposite side of the bodily tissue such that the first optical detector arrangement detects at least a portion of the optical radiation emitted by the first optical source arrangement and the second optical detector arrangement detects at least a portion of the optical radiation emitted by the second optical source arrangement.

Proposed concepts thus aim to provide schemes, solutions concepts, designs, methods, and system pertaining to a PPG sensor system for measuring oxygen saturation of blood within bodily tissues. In particular, embodiments aim to provide a PPG sensor system configured for bidirectional optical radiation sensing by the configuration of first and second optical source arrangements and first and second optical detector arrangements.

Typically, transmission-based PPG sensor systems involve the emission and detection of optical radiation in a single direction across the thickness of the proposed tissue. However, bodily tissue is most commonly not homogeneous, and therefore the ray paths of optical radiation may vary significantly between measurements. Factors that may affect this are the exact type and composition of the bodily tissue and the presence of tissue surface features such as moles and birthmarks. The variation in detected ray paths leads to variation in the region of tissue which the PPG sensor system gathers information about. This may result in errors in the calibration of the PPG sensor system.

The configuration of the PPG sensor of the proposed invention permits for transmissive measurements of optical radiation in two opposite directions across the thickness of a bodily tissue. By combining the information gathered from both these measurements, the impact of inhomogeneity in the bodily tissue and the presence of features on the surface of the bodily tissue on the accuracy of blood oxygen saturation measurements may be reduced. The proposed invention may therefore advantageously result in more accurate and consistent measurements of blood oxygen saturation across different bodily tissues.

In some embodiments, the first optical source arrangement may be configured, in use, to emit optical radiation in a first time period and the second optical source arrangement may be configured, in use, to emit optical radiation in a second time period different form the first time period. Alternating the emission of the optical source arrangements in this way may improve the accuracy of the measured oxygen saturation since the measurement of optical radiation at each of the optical detector arrangements can be clearly attributed to the emission from the corresponding optical source arrangement.

In some embodiments, the first time period and the second time period may not overlap. This may ensure that optical radiation emitted from the first optical source arrangement does not interact with optical radiation emitted form the second optical source arrangement, resulting in more accurate bi-directional measurement of the transmissive ray paths through the bodily tissue.

In some embodiments, the first optical source arrangement may comprise: a first optical source configured to emit optical radiation within a first wavelength range; and a second optical source configured to emit optical radiation within a second wavelength range, wherein the first optical detector arrangement is configured to detect optical radiation within both the first and second wavelength ranges. Some forms of pulse oximetry require the emission of optical radiation of at least two different wavelengths. Optical radiation of different wavelengths traverses different ray paths through the bodily tissue as the optical properties of bodily tissues are wavelength dependent resulting in the two different wavelengths measuring different blood volumes. By ensuring that at least the first optical source arrangement can emit at two different wavelengths, the blood volumes associated with the two different wavelength ranges may be more consistent and have greater overlap. Pulse oximetry measurements may involve calculating a ratio between the detected radiation levels at two different wavelengths. If the volumes associated with each of these measurements do not match, the algorithm that utilizes this measured ratio to determine blood oxygen saturation values becomes inaccurate, introducing calibration errors into the calculation of blood oxygen saturation

In some embodiments, the second optical source arrangement comprises: a third optical source configured to emit optical radiation within the first wavelength range; and a fourth optical source configured to emit optical radiation within the second wavelength range, wherein the second optical detector arrangement is configured to detect optical radiation within both the first and second wavelength ranges. By configuring both optical source arrangements to emit within two different wavelength ranges the accuracy of measured oxygen saturation values may be improved further.

In some embodiments, the first wavelength range comprises red optical wavelengths and the second wavelength range comprises infrared optical wavelengths. Red and infrared optical radiation are particularly useful for pulse oximetry since they have well separated molar absorption coefficients of oxygenated haemoglobin and deoxygenated haemoglobin.

In some embodiments, the first optical source arrangement and the second optical source arrangement are both configured to emit optical radiation within the same wavelength range. Since the measured volume of the tissue is dependent on the wavelength of the radiation, ensuring both the first and second optical source arrangements emit radiation within the same wavelength range may allow for a direct combination of the measured signals, improving the efficiency and accuracy of the PPG sensing.

In some embodiments, the first optical detector arrangement may comprise a plurality of optical detectors configured to detect optical radiation, wherein each of the plurality of optical detectors is configured to detect optical radiation within a different wavelength range. The use of multiple detector components provides a simple means of multi-wavelength detection which may be required for some pulse oximetry applications of the proposed system.

In some embodiments, the first optical source arrangement and the second optical detector arrangement may be configured such that, in use, a portion of the optical radiation emitted by the first optical source arrangement is received at the second optical detector arrangement via a reflective ray path. Optionally, the second optical source arrangement and the first optical detector arrangement are configured such that, in use, a portion of the optical radiation emitted by the second optical source arrangement is received at the first optical detector arrangement via a reflective ray path. Thus, the proposed embodiment may allow for the detection of reflective and transmissive ray paths, the analysis of which may result in more accurate determination of physiological values such as the blood oxygen concentration.

In some embodiments, the first optical source arrangement and the second optical detecting arrangement may form the same component. For instance, an LED may act as both an optical radiation source and an optical radiation detector. Using such components in the proposed invention may save space and help achieve a compact PPG sensor system.

In some embodiments, the first optical source arrangement may be configured to emit first coded optical radiation having a first pattern and the second optical source arrangement may be configured to emit second coded optical radiation having a second pattern different from the first pattern. Using two different forms of coded optical radiation in this way allows for the identification of whether the optical radiation detected at each of the optical detector arrangements originated at the corresponding optical source arrangement. This may result in reduced levels of noise in the measured signals, in particular for the case when the first and second optical source arrangements are configured to emit optical radiation simultaneously.

According to an aspect of the proposed invention, there is provided a pulse oximeter device comprising any of the PPG sensor systems described above.

According to another aspect of the proposed invention, there is provided a system for measuring oxygen saturation of blood within bodily tissue, the system comprising: the pulse oximeter device described above; and a processing arrangement configured to: receive from the pulse oximeter a first optical radiation detection signal describing optical radiation detected by the first optical detector arrangement of the pulse oximeter; receive from the pulse oximeter, a second optical radiation detection signal describing optical radiation detected by the second optical detector arrangement of the pulse oximeter; and determine an oxygen saturation value based on the first and second optical radiation signals.

In some embodiments, the processing arrangement is configured to determine an oxygen saturation value based on the first and second optical radiation signals by: calculating an average of the first and second optical radiation signals; and determining the oxygen saturation value based on the average of the first and second optical radiation signals.

According to yet another aspect of the proposed invention there is provided a method for measuring oxygen saturation of blood within bodily tissue, the method comprising: emitting optical radiation from a first optical source arrangement positioned at a first side of a bodily tissue; detecting at least a portion of the optical radiation emitted by the first optical source arrangement at a first optical detector arrangement on a second side of the bodily tissue wherein the second side is opposite the first side; emitting optical radiation from a second optical source arrangement at the second side of the bodily tissue; and detecting at least a portion of the optical radiation emitted from the second optical source arrangement at a second optical detector arrangement positioned at the first side of the bodily tissue.

Embodiments may be employed in combination with conventional/existing PPG sensing methods and systems. In this way, embodiments may integrate into legacy systems to improve and/or extend their functionality and capabilities. An improved PPG sensor system that allows for the determination of oxygen saturation in blood based on detected optical radiation may therefore be provided by the proposed embodiments.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a simplified illustration of a PPG sensor system according to a proposed embodiment;
Fig. 2 shows a simplified illustration of a PPG sensor system according to another proposed embodiment;
Fig. 3 shows a simplified illustration of a PPG sensor system according to a third proposed embodiment;
Fig. 4 depicts a simplified block diagram of a system for measuring oxygen saturation of blood within bodily tissue according to an aspect of the proposed invention; and
Fig. 5 depicts a simplified flow diagram of a method for operating a PPG sensor system according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The proposed invention provides a system and method for PPG sensing that may be advantageous in reducing calibration errors and inaccuracies in measurements of blood oxygen saturation.

Tissue is most commonly not homogeneous. Optical radiation can travel in different ways and through different blood vessels when entering from one side or another side of the tissue. By directing optical radiation from both sides of a bodily tissue, the proposed invention may be advantageous as some of the effects this inhomogeneity may be mitigated, improving the reliability of measurements taken across different patients and different bodily tissues. Calibration errors may also be introduced into PPG sensor measurements when optical radiation interacts with nail, moles, birthmarks, or other spots on the surface of the bodily tissue. The proposed set-up may also improve accuracy of oxygen saturation measurements in this instance.

Wavelength is a key factor that may affect the blood volume associated with an optical radiation measurement. The optical properties of the bodily tissue vary significantly with wavelength and therefore so will the ray paths of radiation through the bodily tissue. Thus, different wavelengths of optical radiation have different optical paths because of different scattering and absorption behavior of the tissue for different wavelengths. This results in different wavelengths of radiation carrying information from different portions of a bodily tissue. This may introduce calibration defects for multi-wavelength measurements. This effect may be particularly prominent for systems where the optical radiation sources are small and/or the distance between the optical radiation sources and the surface of the bodily tissue is small.

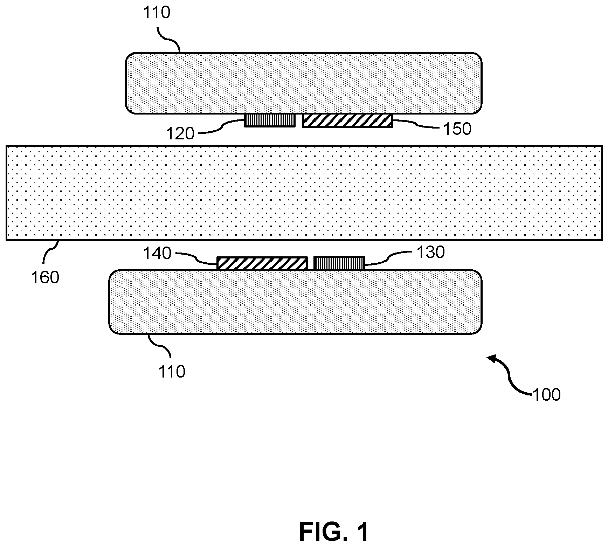

Referring now to Fig. 1, there is depicted a simplified illustration of a PPG sensor system 100 according to a proposed embodiment.

The PPG sensor system 100 comprises a support structure 110 that is configured to receive bodily tissue 160. In particular, the support structure 110 is configured to accommodate a subject's finger.

The PPG sensor system 100 further comprises a first optical source arrangement 120 coupled to the support structure 110 and configured to emit optical radiation. In use, the support structure 110 is configured such that the first optical source arrangement is positioned at a first side of the bodily tissue. The first optical source arrangement is configured to emit optical radiation to the bodily tissue.

A second optical source arrangement 130 is also coupled to the support structure 110. In use, the second optical source arrangement is configured to be positioned at a second side of the bodily tissue, opposite the first side and to emit optical radiation to the bodily tissue.

The PPG sensor system 100 further comprises a first optical detector arrangement 140 and a second optical detector arrangement 150 coupled to the support structure 110. The support structure 110 is configured such that, in use, the first optical detector arrangement 140 is positioned at the second side of the bodily tissue and the second optical detector arrangement 150 is positioned at the first side of the bodily tissue.

The arrangement of the first and second optical source arrangements and the first and second optical detector arrangement is configured such that the first optical detector arrangement detects at least a portion of the optical radiation emitted by the first optical source arrangement and the second optical detector arrangement detects at least a portion of the optical radiation emitted by the second optical source arrangement.

In this particular embodiment the first optical source arrangement is configured, in use, to emit optical radiation in a first time period and the second optical source arrangement is configured, in use to emit optical radiation in a second time period different from the first time period. The first and second time periods in this example do not overlap. Further, in this exemplary embodiment, the first optical source arrangement and the second optical source arrangement are both configured to emit optical radiation within the same wavelength range.

In this way the PPG sensor system 100 is configured to emit pulses of optical radiation first from the first optical source arrangement and then from the second optical source arrangement. Thus, the bodily tissue is subjected to optical radiation passing through its thickness via transmissive ray paths first in a first direction, from the first side of the bodily tissue to the second side of the bodily tissue, and then in a second direction, from the second side of the bodily tissue to the first side. Scattering and absorption effects of optical radiation within the bodily tissue affect the region of the bodily tissue associated with functional information within the measured optical radiation. Since bodily tissues are typically inhomogeneous, the measured region may vary depending on the direction in which optical radiation is transmitted through the tissue. Using bi-directional measurement of radiation (as used in the proposed PPG sensor system 100) may result in greater consistency in the volume of the tissue contributing to functional information in the measured signal across measurements of different patients and different tissues.

The first optical source arrangement 120 and the second optical detector arrangement 150 of the PPG sensor system 100 are configured such that, in use, a portion of the optical radiation emitted by the first optical source arrangement is received at the second optical detector arrangement via a reflective ray path. Similarly, the second optical source arrangement 130 and the first optical detector arrangement 140 are configured such that, in use, a portion of the optical radiation emitted by the second optical source arrangement is received at the first optical detector arrangement via a reflective ray path. Thus, the PPG sensor system 100 is configured such that the optical radiation emitted by the first optical source arrangement 120 is received at the first optical detector arrangement 140 via transmissive ray paths through the bodily tissue 160 and at the second optical detector arrangement 150 via reflective ray paths through the bodily tissue 160. Optical radiation emitted by the second optical source arrangement 130 is received at the second optical detector arrangement 150 via transmissive ray paths and received at the first optical detector arrangement 140 via reflective ray paths. The proposed PPG sensor system 100 therefore combines reflective- and transmissive-based functionalities. This may result in a more accurate determination of blood oxygen saturation from the detected optical radiation.

Fig. 1 shows just one possible configuration of the support structure 110. In other embodiments, the support structure maybe configured differently with any suitable combination of flexible and rigid elements to ensure that in use the first optical source arrangement and the second optical detector arrangement are at the first side of the bodily tissue and the first optical detector arrangement and the second optical source arrangement are at a second opposite side of the bodily tissue. Although the support structure 110 is configured to receive a patient's finger, in alternative embodiments the support structure 110 may be configured to receive other bodily tissue than a finger, such as a portion of an ear lobe, a portion of a nostril, such as the nasal alar, or a portion of a baby's foot.

Although in Fig. 1, the optical source arrangements and the optical detector arrangements are shown as separate components, this need not be the case in other embodiments. In some embodiments, first optical source arrangement and the second optical detector arrangement form the same component, such as for example an LED that is configured to both emit and detect optical radiation. In other embodiments, a similar component is used for the second optical source arrangement and the first optical detector arrangement. This may be advantageous in reducing the size and cost of the PPG sensor system.

Similarly other aspects of the PPG system 100 may be implemented differently in alternative embodiments. Although the PPG system 100 is configured to emit optical radiation in nonoverlapping pulses from the first and second optical sources this need not be the case in all embodiments. In some embodiments, optical radiation may be emitted simultaneously from both source arrangements. In this case, coded optical radiation may be used to aid the identification of the origin of the optical radiation detected at each of the first and second optical detector arrangements. The emission of coded optical radiation involves the emission of optical radiation of a specific predetermined spatial or temporal pattern. In detail, in some embodiments the first optical source arrangement is configured to emit first coded optical radiation having a first pattern and the second optical source arrangement may be configured to emit second coded optical radiation having a second pattern different from the first pattern. Optical radiation incident on the first detector arrangement may have arrived via transmissive ray paths from the first optical source arrangement, or via reflective ray paths from the second optical source arrangement. External sources of optical radiation may also be present. By configuring the first and second optical source arrangements to emit coded optical radiation associated with different patterns, the source of the optical radiation detected at the first detector arrangement can be identified. The advantages of using coded optical radiation equally apply to the second optical detector arrangement.

The components of the PPG sensor system 100 need not be configured for both reflective and transmissive sensing, but instead may be configured (for example by adjusting the relative positions of each of the first and second source arrangements and the first and second detector arrangements) such that only transmissive ray paths are detected at each of the first and second detector arrangements. This may permit easier identification of optical radiation detected at the first and second optical detector arrangements that is emitted at the first and second optical source arrangements, respectively.

Further the first optical source arrangement and the second optical source arrangement need not emit optical radiation within the same optical wavelength range but may emit optical radiation of different wavelengths. This is efficient way of achieving multi-wavelength measurements whilst reducing calibration errors associated with monodirectional optical detection. In this example, filtering at the first and second optical detector arrangements may be used in some embodiments to reduce cross talk between the first optical source arrangement and the second optical detector arrangement, and between the second optical source arrangement and the first optical detector arrangement.

For example, in one embodiment of the present invention, the first optical source arrangement is configured to emit red light, i.e. red optical wavelengths, and the second optical source arrangement is configured to emit infrared radiation, i.e. infrared optical wavelengths. The first optical detector arrangement employs a low pass filter such that the first optical detector detects red light but is insensitive to infrared radiation. The second optical detector arrangement employs a high pass filter such that the second optical detector arrangement detects infrared radiation by is insensitive to red light.

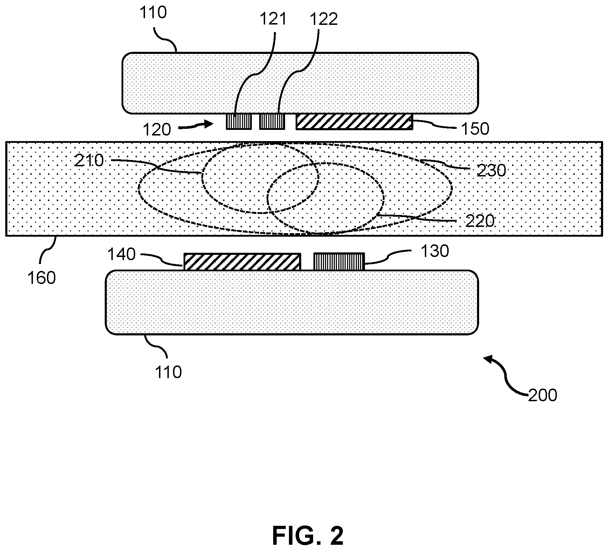

Referring now to Fig. 2, there is depicted a PPG sensor system 200 according to another proposed embodiment.

As in the PPG sensor system 100, the PPG sensor system 200 comprises a support structure 110, a first optical source arrangement 120, a second optical source arrangement 130, a first optical detector arrangement 140, and a second optical detector arrangement 150.

The PPG sensor 200 differs from the PPG sensor system 100 in that the first optical source arrangement 120 comprises two optical sources: a first optical source 121 configured to emit optical radiation within a first wavelength range; and a second optical source 122 configured to emit optical radiation within a second wavelength range. The second optical detector 140 arrangement is further configured to detect optical radiation within both the first and second wavelength ranges. The second optical source arrangement 130 is configured to emit radiation within the first wavelength range.

In this exemplary embodiment, the first wavelength range comprises red optical wavelengths and the second wavelength range comprises infrared optical wavelengths. Red and infrared optical radiation have different scattering behavior within the bodily tissue. Therefore, the different optical wavelengths are absorbed by different regions of the bodily tissue, resulting in different measurements of the blood volume. The blood volume associated with red light emission may be smaller than the blood volume associated with infrared optical radiation as infrared wavelengths undergo lower levels of scattering.

For example, red optical radiation from the first optical source 121 and detected at the first optical detector arrangement 140 may scatter predominantly in a first region 210 of the bodily tissue. Therefore, the detection of red optical radiation at the first optical detector arrangement 140 comprises information predominantly about this first region of the bodily tissue. Detection of red optical radiation at the second optical detector arrangement 150 from the second optical source arrangement 130 may be associated with a similarly sized region of the bodily tissue 220 as depicted in Fig. 2. In contrast, the infrared radiation emission from the second optical source 122 of the first optical source arrangement that is detected at the first optical detector arrangement 130 may be associated with a relatively larger volume 230.

When calculating blood oxygen saturation values from PPG sensor measurements, calibration errors may be introduced when the red and infrared wavelengths do not measure the same blood volumes. Since, in the PPG sensor system 200, red optical radiation is emitted to the tissue from the first optical source arrangement and the second optical source arrangement, the total volumes of the bodily tissue associated with the red optical radiation measurement and the infrared radiation measurements show greater alignment than single source arrangement systems.

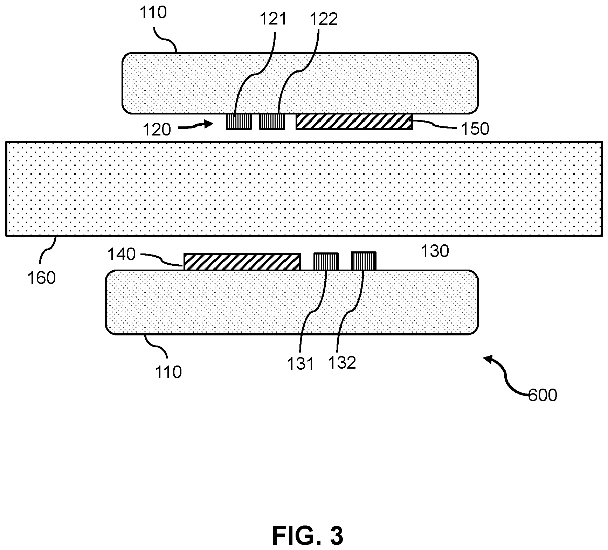

Referring now to Fig. 3, there is depicted a PPG sensor systems 300 according to another proposed embodiment.

The PPG sensor system 300 differs from the PPG sensor system 200 in that the second optical source arrangement comprises third (131) and fourth (132) optical sources. The third optical source 131 is configured to emit optical radiation within the first wavelength range and the fourth optical source 132 is configured to emit optical radiation within the second wavelength range. The second optical detector arrangement 140 of the PPG sensor system 300 is further configured to detect optical radiation within both the first and second wavelength ranges.

The arrangement of the PPG sensor system 300 may be advantageous in that the averaging of detections made by the first and second optical detector arrangements of both wavelengths may result in greater overlap of the associated blood volumes and therefore a more accurate measurement of blood oxygen saturation.

Although the PPG sensory systems 200 and 300 are discussed in the context of red and infrared optical wavelengths, in other embodiments other wavelengths may be used depending on how the measured radiation signals are intended to be processed. In Figs 2 and 3, each of the first and second optical detector arrangements are depicted as single components however, this need not be the case. In some proposed embodiments, each of the first and second optical detector arrangements comprise a plurality of optical detectors configured to detect optical radiation, each of the plurality of optical detectors configured to detect optical radiation within a different wavelength range. Alternatively, or in addition, different filtering elements may be used in combination with the optical detector arrangements of the proposed arrangement such that different portion of the optical detector arrangement are sensitive to optical radiation of different wavelengths. In this way, the detection of optical radiation emitted by optical sources 121, 122, 131, and 133 may be identified based on the wavelength of the optical radiation detected at the first and second optical detector arrangements. This may be advantageous in the case of simultaneous emission from the different optical sources of the first and second optical source arrangements.

Although the PPG sensor systems 200 and 300 are discussed in the context of transmissive-based PPG sensing, it is proposed that in some examples of the invention they are adapted to combine reflective and transmissive-based PPG sensing in a similar way as described for the PPG sensor system 100. Thus, in some examples, the second optical detector arrangement 150 may be configured to measure optical radiation from the first and second optical sources 121 and 122 that traverses the bodily tissue 160 via reflective ray paths, and/or the first optical detector arrangement may be configured to measure optical radiation from the third and fourth optical sources 131 and 132. The ray paths of optical radiation through the bodily tissue 160 are dependent on the wavelength of the optical radiation. Therefore, adapting the PPG sensor systems 200 and 300 for the detection of reflective ray paths may be dependent on the values of the first and second wavelength ranges.

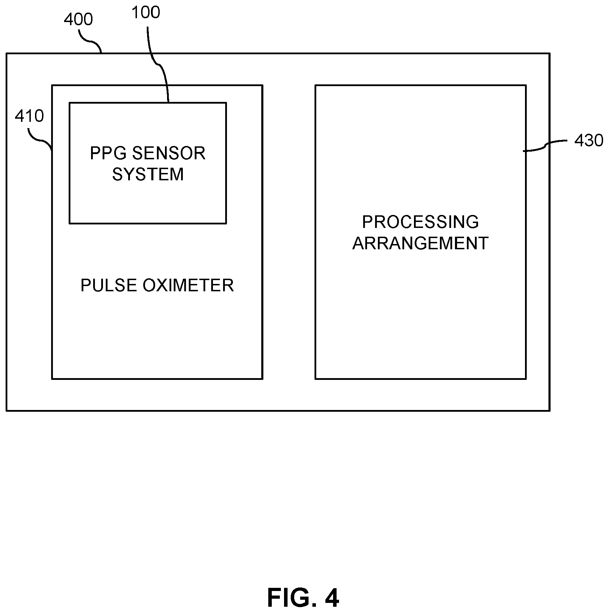

Referring now to Fig. 4, there is depicted a simplified block diagram of a system 400 for measuring oxygen saturation of blood in bodily tissue. The system 400 comprises a pulse oximeter device 410 which houses the PPG sensor system 100. The pulse oximeter device is configured to detect and measure the oxygen saturation levels in the blood. The PPG sensor system 100, as previously described, comprises a support structure, a first optical source arrangement, a second optical source arrangement, a first optical detector arrangement, and a second optical detector arrangement. The components of the PPG sensor system 100 are configured such that, in use, the system receives a bodily tissue and measures transmissive ray paths across the bodily tissue at two opposite sides of the bodily tissue.

The system 400 further comprises a processing arrangement 430. The processing arrangement is configured to: receive from the pulse oximeter a first optical radiation detection signal describing optical radiation detected by the first optical detector arrangement of the pulse oximeter; receive from the pulse oximeter, a second optical radiation detection signal describing optical radiation detected by the second optical detector arrangement of the pulse oximeter; and determine an oxygen saturation value based on the first and second optical radiation signals. As one example, this may comprise taking an average of the first and second optical radiation signals, but other methods may be used to combine the information of the first and second optical radiation signals.

As discussed above, the proposed system makes use of a processing arrangement to perform data processing. The processing arrangement can be implemented in numerous ways, with software and/or hardware, to perform the various functions required. The processing arrangement typically employs one or more microprocessors that may be programmed using software (e.g., microcode) to perform the required functions. The processing arrangement may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing arrangement may be associated with one or more storage media such as volatile and non-volatile computer memory such as RAM, PROM, EPROM, and EEPROM. The storage media may be encoded with one or more programs, that, when executed on one or more processors and/or controllers, perform the required functions. Various storage media may be fixed within a processor or controller or may be transportable, such that the one or more programs stored thereon can be loaded into a processor.

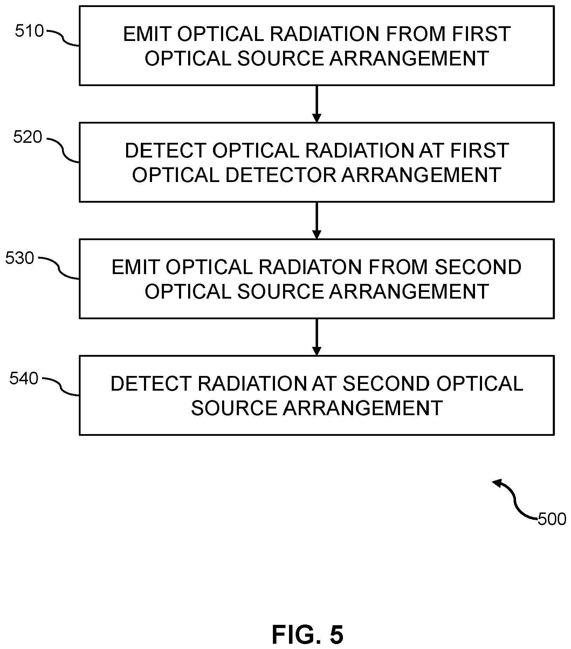

Referring now to Fig. 5, there is depicted a simplified flow diagram of a method 500 for measuring oxygen saturation of blood within bodily tissue.

The method commences with step 510 comprising emitting optical radiation from a first optical source arrangement positioned at a first side of a bodily tissue. Step 520 then comprises detecting at least a portion of the optical radiation emitted by the first optical source arrangement at a first optical detector arrangement on a second side of the bodily tissue, wherein the second side is opposite the first side.

The method then proceeds to step 520 comprising emitting optical radiation from a second optical source arrangement at the second side of the bodily tissue and then to a step 530 of detecting at least a portion of the optical radiation emitted from the second optical source arrangement at a second optical detector arrangement positioned at the first side of the bodily tissue.

Variations to the disclosed embodiments can be understood and effected by those skilled in the art in practicing the claimed invention from a study of the drawings the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If the term "adapted to" is used in the claims or description it is noted the term "adapted to" is intended to be equivalent to the term "configured to". Any reference signs in the claims should not be construed as limiting the scope.

## Claims

1. A PPG sensor system (100) for measuring oxygen saturation of blood within bodily tissue the system comprising:
a support structure (110) configured, in use, to receive bodily tissue;
a first optical source arrangement (120) coupled to the support structure and configured to emit optical radiation;
a second optical source arrangement (130) coupled to the support structure and configured to emit optical radiation;
a first optical detector arrangement (140) coupled to the support structure and configured to detect optical radiation incident on the first optical detector arrangement; and
a second optical detector arrangement (150) coupled to the support structure and configured to detect optical radiation incident on the second optical detector arrangement,
wherein the support structure is configured such that, in use, the first optical source arrangement and the second optical detector arrangement are positioned at a first side of the bodily tissue, and the second optical source arrangement and the first optical detector arrangement are positioned at a second opposite side of the bodily tissue such that the first optical detector arrangement detects at least a portion of the optical radiation emitted by the first optical source arrangement and the second optical detector arrangement detects at least a portion of the optical radiation emitted by the second optical source arrangement.

2. The PPG sensor system of claim 1, wherein the first optical source arrangement (120) is configured, in use, to emit optical radiation in a first time period and the second optical source arrangement is configured, in use, to emit optical radiation in a second time period different from the first time period.

3. The PPG sensory system of claim 2, wherein the first time period and the second time period do not overlap.

4. The PPG sensor system of any preceding claim, wherein the first optical source arrangement (120) comprises:
a first optical source (121) configured to emit optical radiation within a first wavelength range; and
a second optical source (122) configured to emit optical radiation within a second wavelength range, and
wherein the first optical detector arrangement is configured to detect optical radiation within both the first and second wavelength ranges.

5. The PPG sensor system of claim 4, wherein the second optical source arrangement (130) comprises:
a third optical source (131) configured to emit optical radiation within the first wavelength range; and
a fourth optical source (132) configured to emit optical radiation within the second wavelength range, and
wherein the second optical detector arrangement is configured to detect optical radiation within both the first and second wavelength ranges.

6. The PPG sensor system of any of claims 4 and 5, wherein the first wavelength range comprises red optical wavelengths and the second wavelength range comprises infrared optical wavelengths.

7. The PPG sensor system of any preceding claim, wherein the first optical source arrangement (120) and the second optical source arrangement (130) are both configured to emit optical radiation within the same wavelength range.

8. The PPG sensor system of any preceding claim, wherein the first optical detector arrangement (140) comprises a plurality of optical detectors configured to detect optical radiation, wherein each of the plurality of optical detectors is configured to detect optical radiation within a different wavelength range.

9. The PPG sensor system of any preceding claim wherein the first optical source arrangement (120) and the second optical detector arrangement (150) are configured such that, in use, a portion of the optical radiation emitted by the first optical source arrangement is received at the second optical detector arrangement via a reflective ray path, and
optionally wherein the second optical source arrangement (130) and the first optical detector arrangement (140) are configured such that, in use, a portion of the optical radiation emitted by the second optical source arrangement is received at the first optical detector arrangement via a reflective ray path.

10. The PPG sensor system of any preceding claim, wherein the first optical source arrangement (120) and the second optical detector arrangement (150) form the same component.

11. The PPG sensor system of any preceding claim, wherein the first optical source arrangement (120) is configured to emit first coded optical radiation having a first pattern and the second optical source arrangement is configured to emit second coded optical radiation having a second pattern different from the first pattern.

12. A pulse oximeter device (410) comprising the PPG sensor system (100) of any of claims 1-11.

13. A system (400) for measuring oxygen saturation of blood within bodily tissue, the system comprising:
the pulse oximeter (410) device of claim 12; and
a processing arrangement (430) configured to:
receive from the pulse oximeter a first optical radiation detection signal describing optical radiation detected by the first optical detector arrangement of the pulse oximeter;
receive from the pulse oximeter, a second optical radiation detection signal describing optical radiation detected by the second optical detector arrangement of the pulse oximeter; and
determine an oxygen saturation value based on the first and second optical radiation signals.

14. The system of claim 13, wherein determining an oxygen saturation value based on the first and second optical radiation signals comprises:
calculating an average of the first and second optical radiation signals; and
determining the oxygen saturation value based on the average of the first and second optical radiation signals.

15. A method for measuring oxygen saturation of blood within bodily tissue, the method comprising:
emitting optical radiation from a first optical source arrangement positioned at a first side of a bodily tissue;
detecting at least a portion of the optical radiation emitted by the first optical source arrangement at a first optical detector arrangement on a second side of the bodily tissue, wherein the second side is opposite the first side;
emitting optical radiation from a second optical source arrangement at the second side of the bodily tissue; and
detecting at least a portion of the optical radiation emitted from the second optical source arrangement at a second optical detector arrangement positioned at the first side of the bodily tissue.
